# EUROPEAN PATENT APPLICATION

(11) **EP 2 418 221 A1**
(43) Date of publication of application: **15.02.2012**
(21) Application number: 10290442.2
(22) Date of filing: 10.08.2010
(51) Int. Cl.: C07K 16/28, A61K 39/00

(54) **Specific antibodies to G protein-coupled receptors and method for producing the same**

(71) Applicant: Centre National de la Recherche Scientifique CNRS, 75016 Paris (FR)
(72) Inventor: Talmont, Franck, 31140 Pechbonnieu (FR); Dietrich, Gilles, 31470 Fonsorbes (FR)
(74) Representative: Vial, Lionel

(57) **Abstract**

The present invention relates to a method for producing an anti-G Protein-Coupled Receptor (GPCR) antibody or a cell producing an anti-GPCR antibody, comprising a step of recovering the antibody or the cell producing the antibody from a biological sample obtained from an animal which has been immunized with an immunogenic composition prepared from an unfolded GPCR.

## Description

### Field of the invention

The present invention relates to specific anti-G protein-coupled receptor (GPCR) antibodies and methods for obtaining such antibodies.

### Background of the invention

G protein-coupled receptors (GPCRs) are involved in many key biological processes such as smell, taste and vision. Based on the sequence of the human genome, 60% of the 800 GPCRs that have been identified belong to the so-called olfactory or sensory receptors. The remaining 40 % are classified in five main families under the GRAFS system (Glutamate, Rhodopsin, Adhesion, Frizzled/taste2 and Secretin) (Bjarnadottir et al. (2006) Genomics 88:263-273; Schioth & Fredriksson (2005) Gen. Comp. Endocrinol. 142:94-101).

Given their role in a number of physiological life-supporting processes, GPCRs are becoming the most frequent targets for development of new therapeutic drugs. Indeed, nearly half of the drugs marketed by the pharmaceutical industry target this particular class of receptors. The beneficial effect of GPCR-targeting drugs has been reported in a broad spectrum of human pathologies, including cardiovascular and gastrointestinal diseases, central nervous system dysfunction, immune disorders and cancer (Klabunde & Hessler (2002) Chembiochem 3:928-944). Thus, improvement of drug efficacy as well as new drug design requires a better understanding of the functional properties of these receptors.

Antibodies specific for GPCRs are powerful tools which are particularly helpful to analyze the anatomical localization or to describe biochemical and biological properties of the GPCRs (Gupta & Devi (2006) Mt Sinai J. Med. 73:673-681; Gupta et al. (2008) Comb. Chem. High Throughput Screen. 11:463-467). The antibodies can be used as detecting antibodies, to reveal cell surface expression of GPCRs *in vitro,* either on living cells or on membrane extracts, as well *as in situ* on fixed tissue sections. Specific anti-GPCR antibodies may also be helpful to specifically purify receptors (Sarramegna et al. (2006) Cell. Mol. Life Sci. 63:1149-1164), to characterize receptor dimers (Harrison & van der Graaf (2006) J. Pharmacol. Toxicol. Methods 54:26-35) and to identify their protein partners (Bockaert et al. (2004) Curr. Opin. Drug Discov. Devel. 7:649-657) or to help GPCR stabilization for crystallography (Day et al. (2007) Nat. Methods 4:927-929). In addition, depending on their agonistic or antagonistic properties, they may be instrumental in triggering or neutralizing peripheral receptor mediated effects *in vivo* (Mace et al. (1999) J. Biol. Chem. 274:20079-20082; Mace et al. (2002) Blood 100:3261-3268; Macé et al. (1999) Eur. J. Immunol. 29:997-1003; Macé, et al. (2001) Biochem. Biophys. Res. Commun. 280:1142-1147*.).*

Currently, the main strategy to generate antibodies against GPCRs is to inject synthetic peptides, comprising one or multiple epitopes, usually corresponding to amino-acid sequences located within the amino- or carboxy-terminal segment of the receptors, conjugated to carrier proteins (Zhang et al. (2006) Brain Res. 1099:33-43; Gupta et al. (2007) J. Biol. Chem. 282:5116-5124; Peter et al. (2004) J. Biol. Chem. 279:55697-55706; Gomes et al. (1999) FEBS Lett. 456:126-130), to animals. This method is the preferred method used by bioscience companies and academic laboratories because of its simplicity.

Nevertheless, despite the use of predictive algorithms (Mackrill (2004) Methods Mol. Biol. 259:47-65) for designing putative immunogenic synthetic peptides, a lack of selectivity is observed for such generated antibodies, which display non-specific cross-reactivity with other plasma membrane proteins. In this regard, a recent report highlights that lack of selectivity appears to be the rule rather than the exception for antibodies against G-protein coupled receptors obtained in this manner (Michel et al. (2009) Naunyn Schmiedebergs Arch. Pharmacol. 379:385-388).

Numerous other strategies are, however, available for obtaining antibodies against a particular GPCR. These strategies are based on the hypothesis that the availability of an active and well folded full size receptor is essential for the generation of specific antibodies. Thus, the use of purified, folded and active receptors in detergents (Mancia et al. (2007) Proc. Natl. Acad. Sci. USA 104:4303-4308; Niebauer et al. (2006) J. Recept. Signal. Transduct. Res. 26:395-415; Chapot et al. (1989) Hybridoma 8:535-543) or in liposomes (Day et al. (2007) Nat. Methods 4:927-929; Jorissen et al. (2009) BMC Cancer 9:200), the injection of GPCR cell transductants (Goetzl et al. (2004) Immunol. Lett. 93:63-69; Ji et al. (2007) Antiviral Res. 74:125-137) or genetic immunisation (Fujimoto et al. (2009) Hum. Antibodies 18:75-80; Adriouch et al. (2005) Cell Immunol. 236:72-77; Moller et al. (2007) Purinergic Signal 3:359-366) were shown to induce the generation of specific antibodies.

However, the latter strategies are time-consuming and labor intensive. Indeed, considering the difficulties to obtain specific antibodies (polyclonal or monoclonal) using the "fully active and folded receptor" strategy, the peptide strategy is still used by many despite its low level of success in generating specific antibodies.

Thus, it is an object of the present invention to provide specific anti-GPCR antibodies overcoming the above limitations and methods for obtaining them.

### Summary of the invention

The present invention arises from the unexpected fmding, by the inventors, that antibodies specific to G protein-coupled receptors (GPCRs) could be elicited against an immunogenic composition prepared from unfolded GPCR, i.e. that a well-folded GPCR is not required for the production of specific anti-GPCR antibodies.

Advantageously, numerous methods which can be easily implemented are known in the art for obtaining important quantities of unfolded GPCR.

Without wishing to be bound to a particular theory, the inventors believe that, upon administration to an animal, unfolded GPCR, optionally subjected to treatments beforehand, may unexpectedly recover some secondary and/or tertiary structures found in native GPCR, thereby forming epitopes against which antibodies may be elicited.

The present invention thus relates to a method for producing an anti-G Protein-Coupled Receptor (GPCR) antibody or a cell producing an anti-GPCR antibody, comprising a step of recovering the antibody or the cell producing the antibody from a biological sample obtained from an animal which has been immunized with an immunogenic composition prepared from an unfolded GPCR.

The present invention also relates to a method for producing an anti-G Protein-Coupled Receptor (GPCR) antibody or a cell producing an anti-GPCR antibody, comprising:
- immunizing an animal with an immunogenic composition prepared from an unfolded GPCR;
- optionally sacrificing the animal; and
- recovering the antibody or the cell producing the antibody from the animal.

The present invention also relates to an anti-G Protein-Coupled Receptor (GPCR) antibody or a cell producing an anti-GPCR antibody obtainable by the above-defined method.

The present invention further relates to an immunogenic composition prepared from an unfolded G Protein-Coupled Receptor (GPCR) for use in the immunization of animals.

The present invention also relates to the use of an unfolded G Protein-Coupled Receptor (GPCR) for the manufacture of an immunogenic composition.

### Detailed description of the invention

G Protein-Coupled Receptors (GPCRs), also named Serpentines or seven-transmembrane domain receptors, are well known to one of skill in the art. They are notably defined in Bjarnadottir et al. (2006) Genomics 88:263-273 and in Schioth & Fredriksson (2005) Gen. Comp. Endocrinol. 142:94-101.

A GPCR according to the invention may be of any kind. In particular, the GPCR of the invention may be an animal, a plant, or a microorganism GPCR. Examples of microorganism GPCRs notably include yeast GPCRs. Examples of animal GPCRs according to the invention include mammalian GPCRs, such as a rat, mouse or human GPCRs, or insect GPCRs, such as drosophila or bee GPCRs. It is however preferred that the GPCR according to the invention is a human GPCR (also denoted hGPCR).

Preferably, the GPCR according to the invention belongs to the A, B, C, D, E or F GPCR class, to the ocular albinism protein family, to the vomeronasal receptor family, to the taste receptor T2R family, or to the orphan GPCR family. These classes and families are well-known to one of skill in the art and are notably defined in the GLIDA database, available from http://pharminfo.pharm.kyoto-u.ac.ip/services/glida/ and described in Okuno et al. (2008) Nucleic Acids Research 36(Database issue):D907-D912. Class A is also known as the rhodopsin-like family, class B as the secretin-like family, class C as the metabotropic glutamate / pheromone family, class D as the fungal pheromone family, class E as the cAMP receptor family, and class F as the frizzled / smoothened family.

Preferably also, the GPCR according to the invention is an opioid receptor, such as a δ-opioid receptor, a µ-opioid receptor, a κ-opioid receptor, and an ORL receptor to nociceptin, or an anti-opioid receptor, such as a neuropeptide FF 1 (NPFF₁) and a neuropeptide FF 2 (NPFF₂) receptor. Even more preferably, the GPCR according to the invention is selected from the group consisting in the µ-opioid receptor, the κ-opioid receptor, and the neuropeptide FF 2 (NPFF₂) receptor.

The µ-opioid receptor, also denoted mu-opioid receptor, is well known to one of skill in the art. Preferably, the µ-opioid receptor is the human µ-opioid receptor, which sequence is notably represented by Genbank Accession number NP_000905.

The κ-opioid receptor, also denoted kappa-opioid receptor, is well known to one of skill in the art. Preferably, the κ-opioid receptor is the human κ-opioid receptor, which sequence is notably represented by Genbank Accession number NP_000903.

The neuropeptide FF 2 (NPFF₂) receptor is well known to one of skill in the art. Preferably, the NPFF₂ receptor is the human NPFF₂ receptor, which sequence is notably represented by Genbank Accession number NP_444264.1.

As intended herein, the term "G protein-coupled receptor (GPCR)" encompasses both the full length GPCR as well as fragments of the GPCR. Preferably, the fragments of the GPCR according to the invention are such that they have at least 80%, more preferably at least 90% and most preferably at least 95% of the length of the full length GPCR. Preferably also, the fragments of the GPCR according to the invention comprise at least 100 amino acids, more preferably at least 200 amino acids, and most preferably at least 300 amino acids.

Besides, as will be clear to one of skill in the art, the GPCR according to the invention may harbor one or more modifications with respect to the corresponding native GPCR, i.e. the GPCR as found in cells or tissues of non-transgenic organisms. In particular, the GPCR of the invention may be linked to other compounds, in particular peptides or proteins, such as purification tags, haptens or immunogenic protein moieties, for instance. By way of example, the GPCR of the invention may thus be linked to a Histine tag or to cMyc. In addition, the sequence of the GPCR may be modified with respect to its native sequence, by substitution, deletion or addition of at least one amino acid, in particular for improving the recombinant production of GPCR or for favoring the appending of the above-mentioned compounds. The substitution, deletion or addition of at least one amino acid should preferably be such that specific antibodies directed against GPCR may still be obtained. Besides, it is preferred that the sequence of a modified GPCR according to the invention, or a fragment thereof, presents more than 90%, more preferably more than 95%, identity with the corresponding native sequence, or the corresponding native fragment thereof.

As intended herein, the expression "unfolded G protein-coupled receptor (GPCR)" relates to a GPCR having lost its tertiary or secondary structure, in partiality or in totality, with respect to the corresponding GPCR in its natural setting, i.e. a folded GPCR, in particular an active folded GPCR.

Preferably, the unfolded GPCR according to the invention binds to a specific ligand thereof with an affinity lower than that of the corresponding folded GPCR, in particular the active folded GPCR. Thus, the unfolded GPCR according to the invention more preferably binds to a specific ligand thereof with an affinity equal to or less than 90%, 80%, 70%, 60%, 50%, 40%, 30%, 20%, 10%, or 0% of the affinity of the corresponding folded GPCR, in particular the active folded GPCR, for the specific ligand. As will be clear to one of skill in the art, the affinity of the folded and unfolded GPCR for the specific ligand is determined under the same experimental conditions. Besides, methods for determining the affinity of a GPCR for a specific ligand are well known to one of skill in the art and are notably described in Strange et al. (2008) Br. J. Pharmacol. 153:1353-63, and Zheng et al. (2010) IUBMB Life 62:112-119.

It is also preferred that the unfolded GPCR according to the invention yields a lower level of G protein activation, in the presence of an agonist of the GPCR, than that of the corresponding folded GPCR, in particular the active folded GPCR. Thus, the unfolded GPCR according to the invention more preferably yields a level of G protein activation in the presence of an agonist of the GPCR which is equal to or less than 90%, 80%, 70%, 60%, 50%, 40%, 30%, 20%, 10%, or 0% of G protein activation yielded by the corresponding folded GPCR, in particular the active folded GPCR. As will be clear to one of skill in the art, the level of G protein activation yielded by the folded and unfolded GPCR, in the presence of the agonist, is determined under the same experimental conditions. Besides, methods for determining the level of G protein activation are well known to one of skill in the art and notably involve GTPγS (*i.e*. guanosine 5'-O-[gamma-thio]triphosphate) as is, for instance, described by Harrison & Traynor (2003) Life Sciences 74:489-508.

Similarly, it is also preferred that the unfolded GPCR according to the invention yields a lower level of second messenger response (e.g. cAMP, Ca²⁺), measured as a variation (*i.e.* an increase or a decrease) in a second messenger concentration, in particular an intracellular concentration, in the presence of an agonist of the GPCR, than that of the corresponding folded GPCR, in particular the active folded GPCR. Thus, the unfolded GPCR according to the invention more preferably yields a level of second messenger response in the presence of an agonist of the GPCR which is equal to or less than 90%, 80%, 70%, 60%, 50%, 40%, 30%, 20%, 10%, or 0% of the second messenger response yielded by the corresponding folded GPCR, in particular the active folded GPCR. As will be clear to one of skill in the art, the level of second messenger response yielded by the folded and unfolded GPCR, in the presence of the agonist, is determined under the same experimental conditions. Methods for determining the level of second messenger response are well known to one of skill in the art.

Preferably also, the unfolded GPCR according to the invention is notably such that less than 50%, more preferably less than 25%, and most preferably less than 10%, of the peptide chain which constitutes the GPCR present a secondary and/or tertiary structure; in this regard, the unfolded GPCR of the invention may notably present no secondary and/or tertiary structures. As intended herein, the expression "unfolded GPCR" and "denatured GPCR" are considered equivalent. The presence of secondary or tertiary structures in a GPCR can be assessed by numerous methods well known to one of skill in the art, such as circular dichroism for instance. As intended herein a "specific ligand" of a GPCR relates to any ligand liable to bind to the receptor site of the GPCR and in particular to induce the activation to the G protein coupled to the GPCR.

An unfolded GPCR of the invention may notably be obtained by contacting a GPCR composition with a detergent. Detergents according to the invention suitable for obtaining an unfolded GPCR according to the invention are well known to one of skill in the art and may be of any type, such as anionic detegents, for example sodium dodecyl sulfate (SDS), cationic detegents, zwitterionic detergents and neutral or non-ionic detergents, for instance Tween. However, it is preferred that the detergent according to the invention is an anionic detergent. Most preferably, the detergent according to the invention is SDS, in particular at a concentration of at least 0.02%, more particularly at a concentration of at least 0.05%, even more particularly at a concentration of at least 0.1%, and preferably at concentration of 2% at the most.

In a preferred embodiment, the unfolded GPCR of the invention is obtained by further contacting the GPCR composition with a protein denaturant. Protein denaturants according to the invention are well known to one of skill in the art and may be of any type. However, it is preferred that the protein denaturant according to the invention is selected from the group consisting of urea, and guanidinium salts, such as guanidinium chloride. Most preferably, the protein denaturant is urea, in particular at a concentration of at least 1 M, more particularly of at least 5 M and even more particularly of at least 8M. Preferably, the protein denaturant is removed from the GPCR composition after unfolded GPCR is obtained. Removing a protein denaturant may be carried out by numerous methods well known in the art, such as dialysis of the GPCR composition against a protein denaturant-free solvent or extracting the GPCR from the composition and diluting it in a protein-denaturant free solvent.

In another preferred embodiment, the unfolded GPCR of the invention is obtained by further contacting the GPCR composition with a disulfide reducing agent. Disulfide reducing agents according to the invention are well known to one of skill in the art and notably encompass β-mercaptoethanol and dithiotreitol (DTT). Preferably, the disulfide reducing agent of the invention is β-mercaptoethanol, in particular at a concentration of at least 5 mM, more particularly at a concentration of at least 15 mM, and even more particularly at a concentration of at least 20 mM.

As will be clear to one of skill in the art, other compounds may further be present when contacting the GPCR composition with the detergent, in particular for stabilizing the GPCR, such as pH buffers or salts. Thus, by way of example, the GPCR composition may be contacted with an aqueous solution comprising or consisting of 100 mM NaH₂PO₄ 10 mM Tris-HCl, 20 mM β-mercaptoethanol, 8M urea, 0.1% sodium dodecyl sulfate (SDS), pH 8.

As intended herein, the expression "GPCR composition" relates to any composition comprising a GPCR. The GPCR composition of the invention can thus be obtained from any source, in particular from animal tissues, in particular non-transgenic animal tissues. However, it is preferred that the GPCR composition of the invention is produced by a recombinant GPCR-expressing microbial strain. As intended herein the expression "recombinant GPCR-expressing microbial strain" encompasses any cell type likely to be propagated in culture, in particular in a liquid culture, such as animal cells, in particular human, cells, bacterial cells, or fungal cells. Preferably, the microbial strain of the invention is a yeast strain, more particularly a *Pichia pastoris* strain.

Treatments for obtaining a GPCR-composition from a recombinant GPCR-expressing microbial strain are well known to one of skill in the art, and are notably illustrated for *Pichia pastoris* in the following non-limiting Example. Usually, the GPCR can be obtained from a protein extract of a culture of the GPCR-expressing microbial strain and be further concentrated and purified through the application of routine techniques. In this regard, the GPCR expressed by the recombinant GPCR-expressing microbial strain may comprise a purification tag, such as a polyhistidine tag.

As intended herein, an "immunogenic composition" relates to any composition suitable to be administered to an animal, in particular in view of obtaining antibodies directed against one or more constituents of the immunogenic composition.

As will be clear to one of skill in the art, the immunogenic composition of the invention may be prepared either directly or indirectly from unfolded GPCR according to the invention. Besides, the immunogenic composition of the invention is preferably prepared from unfolded GPCR in the absence of a protein denaturant according to the invention. Preferably also, the immunogenic composition of the invention is prepared from unfolded GPCR in the presence of a detergent according to the invention, in particular SDS. Besides, it is further preferred that the immunogenic composition according to the invention does not comprise completely folded, in particular active, GPCR.

Where the immunogenic composition of the invention is prepared indirectly from unfolded GPCR, one or more treatments may be applied to unfolded GPCR before it is used for immunizing the animal. Thus, the immunogenic composition may be prepared from lyophilizated unfolded GPCR, in particular after the detergent has been removed from the GPCR composition. Lyophilization of unfolded GPCR may be carried out by numerous methods well knonw to one of skill in the art, such as freeze-drying for instance. In addition, removing the detergent from the GPCR composition may also be carried out by numerous methods well known in the art, such as dialysis of the GPCR composition against a detergent-free solvent, such as water, or extracting the GPCR from the composition and diluting it in a detergent-free solvent, such as water.

Besides, in a preferred embodiment, the immunogenic composition of the invention may be prepared from unfolded GPCR mixed with a composition comprising at least one amphiphile compound and/or hydrophobic compound. As intended herein an amphiphile compound relates to any compound comprising both a hydrophobic and an hydrophilic moiety; such compounds, which notably encompass detergents, are well known to one of skill in the art. Preferably, the amphiphile compound of the invention is D-dianhydromannitol monooleate. Preferably also, the composition comprising an hydrophobic compound according to the invention may comprise paraffin oil. In the latter case the hydrophobic compound is preferably an alkane, more preferably an n-alkane or linear alkane, in particular comprising from 10 to 50 carbon atoms. More preferably, the composition comprising at least one amphiphile compound and/or hydrophobic compound according to the invention is Freund's adjuvant.

Freund's adjuvant is well known to one of skill in the art, it is notably described in Stills (2005) ILAR Journal 46:280-293. Freund's adjuvant notably comprises a suspension of desiccated *Mycobacterium butyricum* in a mixture of paraffin oil, in particular constituted of linear alkanes or n-alkanes, and an emulsifying agent, D-dianhydromannitol monooleate. Freund's adjuvant according to the invention may be complete, i.e. with desiccated *Mycobacterium butyricum,* or incomplete, i.e. without desiccated *Mycobacterium butyricum.*

Thus, in another preferred embodiment, the immunogenic composition of the invention may be prepared from unfolded GPCR mixed with an adjuvant composition. Adjuvant compositions are well known to one of skill in the art and are notably described in Stills (2005) *ILAR Journal* **46**:280-293.

Without wishing to be bound to a particular theory, the inventors believe that that n-alkanes (paraffin oil) and D-dianhydromannitol monooleate (emulsifying agent) within Freund's adjuvant may be useful to partially refold the GPCRs by mimicking molecular interactions of the lipid bilayer cell membrane with GPCRs.

In addition, the immunogenic composition of the invention may further comprise one or more pharmaceutically acceptable carriers.

As intended herein "immunizing" an animal according to the invention relates to administering an immunogenic composition to an animal. Any administration route suitable for eliciting antibodies or antibody-producing cells may be used for immunizing an animal according to the invention. Such routes are well known to one skilled in the art. In particular, the immunogenic composition of the invention may be administered by implantation or through the intramuscular, intraperitoneal, subcutaneous, transdermal, transcutaneous, intravenous, or intrathecal routes.

Preferably, the animal according to the invention is a non-human animal, more preferably a non-human primate, such as an ape or a monkey, a camelid, a horse, a donkey, a swine, a bovine, a sheep, a goat, a canine, a feline, a rabbit, a rodent, such as a rat, a mouse, a hamster, or a guinea pig, a chicken, a turkey or a duck. In certain embodiments of the invention, the animal according to the invention may also be a human.

Numerous methods are known in the art for recovering antibodies or antibody-producing cells from an animal or from a biological sample obtained from an animal. Preferably, the anti-GPCR antibody-producing cells are recovered from secondary lymphoid organs, such as spleen, lymph nodes and mucosa-associated tissues, from the animal immunized according to the invention. Preferably, the anti-GPCR antibodies are recovered from humors or bodily fluids, such as blood, plasma, or serum, from the animal immunized according to the invention.

As will be clear to one of skill in the art, antibodies recovered according to the invention are preferably polyclonal antibodies. These antibodies may be further purified and concentrated. The recovered antibody-producing cells are notably useful for producing monoclonal antibodies, either through fusion with immortalized cell types or by isolating the antibody coding sequences. Besides, as will also be clear to one of skill in the art, the anti-GPCR antibodies according to the invention are preferably specific anti-GPCR antibodies. Specific antibodies to a GPCR preferably present essentially no-cross reaction towards other proteins liable to be found in biological membranes harboring the GPCR or towards unrelated GPCR. As intended herein a GPCR unrelated to a GPCR against which a specific antibody is directed presents preferably less than 70%, more preferably less than 80%, and most preferably less than 90% sequence identity with the GPCR against which a specific antibody is directed.

The invention will be further illustrated by the following, non-limiting, Example and Figures.

### Description of the figures

### Figures 1, 2, 3, 4, 5 and 6: Solubilisation and purification analysis of human G-protein coupled receptors.

Receptors were eluted from an immobilized nickel affinity column with imidazole, analysed after SDS-PAGE by silver nitrate staining and Western blotting using anti cmyc antibodies.
Figures 1 and 2 respectively represent a silver nitrate staining of the 300 mM imidazole purification fraction of human κ-opioid receptor (hKOR) and a Western blot of purified human κ-opioid receptor (hKOR).
Figures 3 and 4 respectively represent a silver nitrate staining of the 300 mM imidazole purification fraction of human NPFF2R (hNPFF₂) and a Western blot of purified human NPFF2R (hNPFF₂).
Figures 5 and 6 respectively represent a silver nitrate staining of the 300 mM imidazole purification fraction of human µ-opioid receptor (hMOR) and a Western blot of purified human µ-opioid receptor (hMOR).

### Figures 7, 8 and 9: Western blot analysis of mice anti-sera

The specificity of the anti-sera generated against the GPCRs was assessed on CHO cells expressing GPCRs *vs*. wild-type CHO cells.

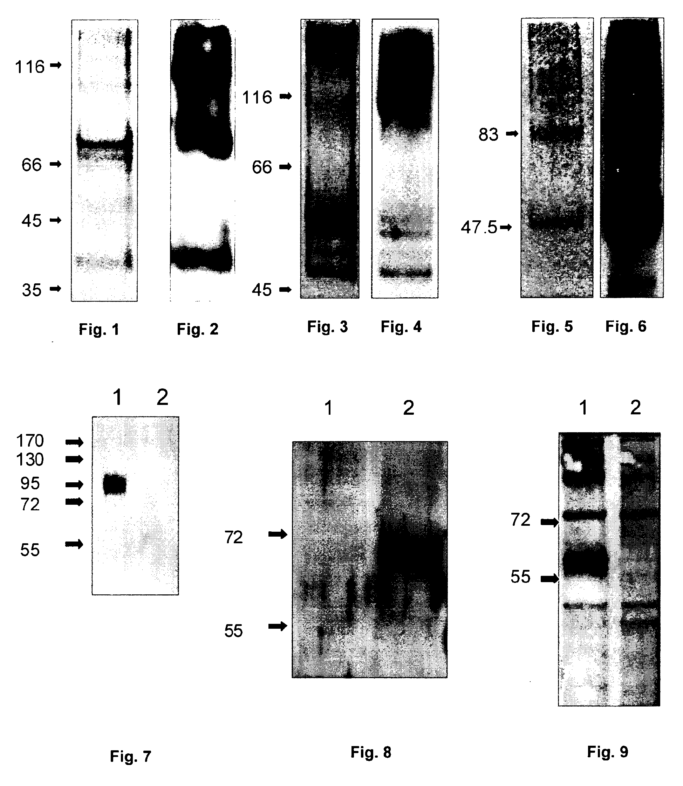

Figure 7 represents the detection of hNPFF₂R in membranes of hNPFF₂R-expressing CHO-K1 cells (lane 1) *vs.* in membranes of wild-type CHO cells (lane 2) at 1/4000 serum dilution.
Figure 8 represents the detection of hµ-opioid receptor (hMOR) in membranes of hµ-opioid-expressing CHO-K1 cells (lane 2) *vs.* in membranes of wild-type CHO cells (lane 1) at 1/1000 serum dilution.
Figure 9 represents the detection of hκ-opioid receptor (hKOR) in membranes of hκ-opioid receptor-expressing CHO-K1 cells (lane 1) vs. in membranes of wild-type CHO cells (lane 2) at 1/1000 serum dilution.

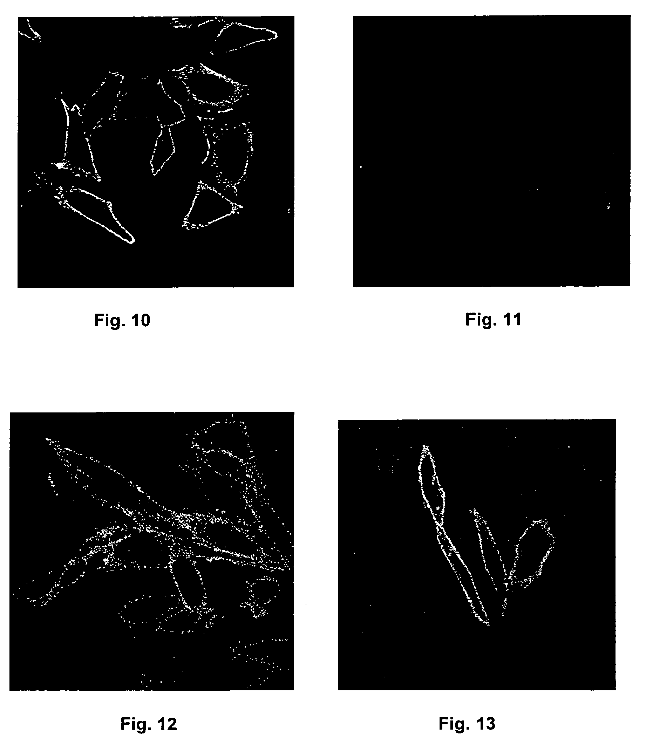

### Figures 10, 11, 12, and 13: Immunofluorescence microscopy detection of human GPCRs in CHO cells by immune sera

Immunofluorescence microscopy detection of human GPCRs was carried out after fixation and permeabilization of CHO cells stably expressing human GPCRs. The cells were incubated with immune sera (1:100) and subsequently with Alexa 488 anti-mouse secondary antibody (1:400).
Figure 10 shows that the fluorescence emitted by CHO cells expressing hNPFF₂R is specifically localized in the plasma membrane. Propidium iodide staining reveals the nuclei of the cells.
Figure 11 shows that no specific fluorescence staining appears using anti-hNPFF₂ antibodies on wild type CHO cells. Propidium iodide staining reveals the nuclei of the cells.
Figure 12 shows cell membrane staining of hκ-opioid receptor-expressing CHO cells.
Figure 13 shows cell membrane staining of hµ-opioid receptor-expressing CHO cells.

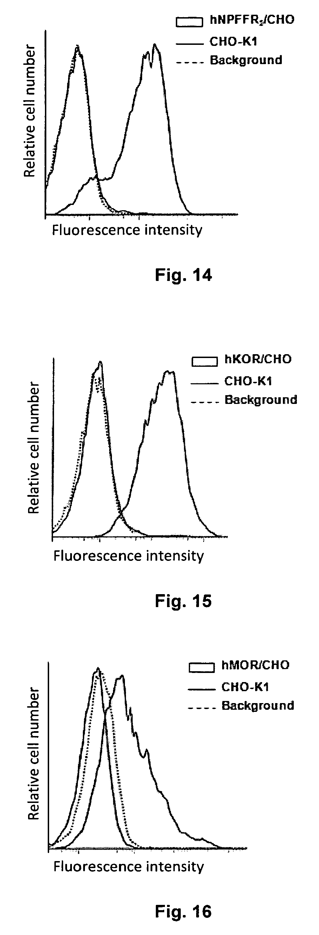

### Figures 14, 15, and 16: Cytofluorometric assessment of polyclonal antibodies binding to GPCR

Figures 14-16 represent the binding of IgG immune serum to either wild-type CHO-K1 cells or CHO-K1 cells expressing hNPFF₂R (Figure 14), hKOR (Figure 15) and hMOR (Figure 16).

GPCR-expressing CHO cells (grey histogram) and CHO-K1 cells (open histogram) were incubated with immune sera for 30 min at 4°C. Bound IgG were then revealed with biotin-conjugated goat anti-mouse antibodies followed by an additional incubation with allophycocyanin-labeled streptavidin. Backgrounds (dotted line) correspond to GPCR-expressing CHO cells or wild-type CHO-K1 cells stained with serum IgG from non-immunized mouse.

### Example

### Methods

### Preparation of recombinant G-protein coupled receptors

GPCR-cmyc-6his plasmid constructs (hMOR-cmyc-6his, hKOR-cmyc-6his, hNPFF₂R-cmyc-6his) as well as the experimental procedures used to overexpress receptors in *Pichia. pastoris* are known in the art and have been notably described in Muller et al. (2008) J. Membr. Biol. 223:49-57, Sarramegna et al. (2002) Protein Expr. Purif. 24:212-220, Sarramegna et al. (2005) Protein Expr. Purif. 43:85-93, and Sarramegna et al. (2002) J. Biotechnol. 99:23-39.

GPCR-enriched fractions were prepared as previously described in Sarramegna et al. (2005) Protein Expr. Purif. 43:85-93. Briefly, after induction of GPCR expression with 0.5% methanol (v/v), GPCR-expressing yeast cells were harvested and broken with glass beads in a breaking buffer (10 mM Tris-HCl, pH 7.5) containing a protease inhibitor cocktail (benzamidine 20 µg/ml, pepstatinA 1 µg/ml, leupeptin 1 µg/ml, antipain 1 µg/ml, aprotinin 1 µg/ml) for 30 min at 4°C. Cell lysate was centrifuged at 1000 g for 15 min to remove unbroken cells and particulate matters. Supernatant was then centrifuged at 10,000 g for 30 min and the pellet containing the crude GPCR-containing fraction was stored in the breaking buffer at -20°C. Receptors were solubilised by diluting crude extracts in the solubilisation buffer (100 mM NaH₂PO₄, 10 mM Tris-HCl, 20 mM β-mercaptoethanol, 8M urea, 0.1% sodium dodecyl sulfate (SDS), pH 8) for 1 h at room temperature with gentle agitation on a rotating wheel. Solubilised receptors were incubated with Ni-chelated Sepharose Fast Flow (GE Healthcare Life Science, Uppsala, Sweden) for 1 h at room temperature. The sepharose resin was then loaded on a plastic column. After two consecutive washes with the solubilisation buffer and with the same buffer without urea and β-mercaptoethanol, Ni-bound GPCR were eluted with a stepwise imidazole gradient buffer (100 mM NaH₂PO₄, 10 mM Tris-HCl, 0.1% SDS, pH 8). Enriched-GPCR samples, as assessed by silver nitrate staining and anti cmyc Western blotting, were extensively dialyzed against pure water and lyophilized. In some experiments, imidazole was eliminated and samples concentrated on a Centricon plus-20 centrifugal filter (Amicon, Millipore Corporation, MA, USA).

### Immunization of mice

Experiments were performed in compliance with the relevant laws and institutional guidelines (INSERM) and were approved by the local ethics committee (Midi-Pyrénées, France). Eight-week-old BALB/c mice (Janvier, Le Genest Saint Isle, France) were injected subcutaneously with 100 µg of purified GPCR (lyophilized or solubilised in 0.1% SDS) emulsified in complete Freund's adjuvant (Difco, Detroit, MI). Two groups of mice (one with two animals and the other with three animals) were injected with human mu-opioid receptors in the lyophilized form and one group containing three animals was injected with the receptor in SDS. For the human NPFF₂R, two groups with three and four animals were injected with the receptor in SDS and one group (3 animals) with the lyophilised receptor. One group of two animals was injected with human k-opioid receptors in SDS. Two subsequent injections two weeks apart were performed with same amounts of GPCR in incomplete Freund's adjuvant. Three days prior to sacrifice, mice were injected intraperitoneally with 200 µg GPCR. Before killing, whole bloods were collected by cardiac punction under general anesthesia of the mice.

### Eukaryotic cell culture and membrane preparation

Wild type CHO-K1 cells and CHO-K1 cells stably expressing human µ-opioid (or mu-opioid) receptor (notably described in Mace et al. (1999) J. Biol. Chem. 274:20079-20082), human κ-opioid (kappa-opioid) receptor (notably described in Mollereau et al. (1999) Mol. Pharmacol. 55:324-331), human NPFF₁ and NPFF₂ receptors (notably described in Mollereau et al. (2002) Eur. J. Pharmacol. 451:245-256) were grown in high glucose DMEM (Invitrogen, Carlsbad, CA) supplemented with 10% fetal calf serum (FCS), 50 µg/ml gentamicine (Invitrogen) and 400 µg/ml geneticin G-418 sulfate to maintain selection (for receptor-expressing cells) at 37°C in 5% CO₂ atmosphere.

For membrane preparation, cells harvested in phosphate buffer saline (PBS), were frozen at -70 °C for at least 1 h and homogenized in 50 mM Tris-HCl, pH 7.5 using a Potter Elvehjem tissue grinder. The homogenate was then centrifuged at 1000 g for 15 min at 4 °C to discard residual cells, nuclei and mitochondria. The membrane fraction was collected upon centrifugation of the supernatant at 100,000 *g* for 30 min at 4 °C. The pellet was resuspended in Tris-HCl 50 mM, pH 7.4 and stored at - 80° C after determination of the protein content.

### Western blot analysis of purified GPCR samples and mouse anti-GPCR sera

Purified samples from *Pichia pastoris* and mouse anti-GPCR sera were analysed by silver nitrate staining, Western blot or dot blot. After SDS-polyacrylamide gel electrophoresis (PAGE) separation, proteins were transferred to a PVDF Immun-Blot membrane (Bio-Rad Laboratories, CA, USA). Purified receptors from *Pichia pastoris* were probed with a monoclonal anti-cmyc antibody (clone 9E10, Sigma, St Louis, Missouri, USA, 1:1000 in PBS-0.5% (w/v)Tween, PBST) and receptors from cell extracts were probed with mice anti-GPCR sera as primary antibodies diluted in 1% BSA PBST. An anti-mouse horseradish peroxidase-coupled antibody (Jackson Immunoresearch, West Grove, PA, USA, 1:10000 in 5% non fat dry milk PBS) was used as a secondary antibody. Immunoreactivity was detected with ECL plus Western Blotting substrate (GE Healthcare Life Science), Uppsala, Sweden) and visualized with CL-XPosure films (Thermo scientific, Waltham, MA, USA). Molecular weight standards were: 116, 66, 45, 35, 25, 18, and 14 kDa (Fermentas, Burlington, Canada) or 170, 130, 95, 72, 55, 43, 34, 26, 17, 10 kDa (Euromedex, Souffelweyersheim, France).

### Cytofluorometric analysis

CHO-K1 or GPCR-expressing CHO cells were incubated with serum from either naïve or GPCR-primed mice for 30 min at 4°C in PBS containing 1% FCS and 2 mM EDTA. After washing, bound IgG were revealed with biotin-conjugated goat anti-mouse IgG antibodies (Jackson Immunoresearch Lab.) followed by an additional incubation with Allophycocyanin-labeled streptavidin (PharMingen, BD Biosciences, San Jose, CA). Data were collected on 10,000 living cells by forward and side scatter intensity on an FACs calibur flow cytometer (Becton Dickinson, Franklin Lakes, NJ), and were subsequently analyzed using the Flow Jo software (Tree Star Inc., Ashland, OR).

### Immunofluorescence microscopy

CHO cells were grown on sterile glass coverslips and fixed with 4% paraformaldehyde for 10 min at room temperature followed by incubation in 50 mM NH₄Cl for 2 min. Cells were then incubated with primary antibody diluted 1:100 in PBS containing 2% FCS for 1 h at room temperature and with Alexa 488 anti-mouse secondary antibody diluted 1:400 in PBS containing 2% FCS for 30 min at room temperature. Cells were then permeabilized in PBS containing 0.2% Triton X-100 and nuclei were stained with 1 µg/mL propidium iodide in PBS also containing 100 µg/ml of RNAseA for 10 min at room temperature. Coverslips were mounted using Vectashield medium (Vector Laboratories, CA, USA). Fluorescence images were taken using an upright laser scanner confocal microscope (Leica TCS SP2, Germany) with x100 oil immersion objective.

### Results

### Antigen preparation

All G-protein receptors were expressed in the methylotrophic yeast *Pichia pastoris,* a way well known to one of skill in the art to generate functional GPCRs in yeast, as notably illustrated by Lundstrom et al. (2006) J. Struct. Funct. Genomics 7:77-91, and purified as a fusion to cmyc and 6-histidine tags (GPCR-cmyc-6his). Besides, it is known that, when expressed in *Pichia pastoris,* the human µ-opioid receptor is mainly found in inclusion bodies-like under an aggregated and misfolded form (Sarramegna et al. (2005) Protein Expr. Purif. 43:85-93). These inclusion bodies-like are sensitive to sodium dodecyl sulphate (SDS), which is an anionic detergent, and is able to completely solubilise the receptor.

Receptors were thus solubilised in a buffer containing 0.1% SDS and 8M urea and purified by means of a 6-histine tag fused to the C-terminus of the receptor and an immobilized-Nickel affinity column (Sarramegna et al. (2002) Protein Expr. Purif. 24:212-220). A step gradient of imidazole in the buffer was used to elute bound receptors from the Nickel-agarose phase. Imadazole-eluted fractions were analysed by silver nitrate staining and anti cmyc Western blotting.

The production of receptor-enriched preparations is illustrated in **Fig.1 and 2** for hNPFF₂ receptor and in **Fig.3 and 4** for κ-opioid receptor and has been already reported for µ-opioid receptor (Sarramegna et al. (2002) Protein Expr. Purif. 24:212-220). Purified receptors showed bands corresponding to monomers on silver stained SDS-PAGE gels with some higher molecular weight bands also present on the gel. These bands were specifically detected by Western blot and were probably oligomers or aggregates of receptors. For human NPFF₂ and human κ-opioid receptors, bands with apparent molecular weight of 47 kDa and 40 kDa, respectively were detected and these values are in accordance with the calculated molecular weight of receptors (**Table 1**) indicating a receptor with no post-transductional modifications. As illustrated in **Fig. 5 and 6**, a same pattern was observed for the human µ-opioid receptors expressed in *Pichia pastoris* (45 kDa for the monomer) (Sarramegna et al. (2002) Protein Expr. Purif. 24:212-220). For all three receptors, oligomers or aggregates are present and may have been generated during the SDS-PAGE or be an indication that GPCRs form oligomers *in vivo.* After purification, samples were depleted of immidazole (to minimize toxicity) and were either dialyzed against pure water and lyophilized (rocky state) or concentrated on a centrifugal filter device in 0.1% SDS (unfolded state).

**Table 1: Human G-protein coupled receptors analysed for their ability to generate specific polyclonal antibodies**

| Receptor name | Gene name | NCBI accession number | AA number | Molecular weight (kDa) |
|---|---|---|---|---|
| µ-opioid | *OPRM1* | NP_000905 | 400 | 44.78 |
| κ-opioid | *OPRK1* | NP_000903 | 380 | 42.65 |
| NPFF₂ | *NPFF2R* | NP 444264.1 | 420 | 48.69 |

| | | | | |
|---|---|---|---|---|
| AA: amino acid | | | | |

### Mice antibody production and characterization

Antibodies specific to human NPFF₂ and µ-opioid receptors were generated by immunizing mice with GPCR preparation containing GPCR either in a rocky state (lyophilized) or under an unfolded-solubilized form (0.1% SDS). The κ-opioid receptor was only injected under the detergent solubilised form. A conventional immunization protocol was followed for the animals using complete and incomplete Freund's adjuvant. Reactivities against GPCRs were first assessed by Western Blot carried out on purified receptors from *Pichia pastoris.*

The specificity of human NPFF₂ (**Fig. 7**), µ-opioid (**Fig. 8**) and κ-opioid (**Fig. 9**) receptors anti-sera was assessed by comparing Western blot patterns obtained with membranes from CHO and GPCR stably expressing CHO cells. Membranes obtained from human NPFF₂R-CHO cells were probed with anti-human NPFF₂R sera over a range of dilutions. The human NPFF₂ receptor anti-serum was able to specifically detect the receptor, which was observed under a unique broad band at 95 kDa, to at least 1:4000 dilution. This apparent molecular weight is higher than expected (~49 kDa, **Table 1**) and may represent a glycosylated form of the receptor, as already observed for many G-protein coupled receptors (as described e.g. by Tansky et al. (2007) Proc. Natl. Acad. Sci. USA 104:10691-10696). In the same manner, the human µ-opioid and κ-opioid receptors were specifically detected at 70 kDa and 60 kDa, respectively, as broad bands, by Western blot at 1:1000 serum dilutions. These molecular weights may also be in favour of a glycosylation of the receptor.

### Immunofluorescence microscopy

Reactivity and specificity of mice antisera were analysed by immunohistochemistry performed on CHO cells stably expressing GPCRs in comparison wild type CHO cells. CHO cells expressing either hNPFF₂R (**Fig.10**), κ-opioid (**Fig.12**) and µ-opioid receptors (**Fig.13**) showed an equivalent pattern of specific membrane labelling whereas wild type CHO cells were unlabelled (**Fig.11**). The use of the secondary antibody alone did not reveal any unspecific fluorescence.

### Cytofluorometric analysis

All three immune sera were also able to recognize receptors in their native conformations at the membrane surface of CHO cells as assessed by confocal microscopy and cytofluorometry. Each IgG immune serum stained CHO cells expressing their respective receptor used as immunogen but not control CHO-K1 cells (**Fig. 10-16**). The control, normal serum IgG from non-immunized mice, bound neither to wild type CHO-K1 cells nor to GPCR-expressing cells.

Thus, the results obtained by the inventors indicate that the use of folded GPCR, i.e. in a three-dimensional active conformation, is not crucial to generate antibodies able to specifically bind to receptors in native conformation. Furthermore, immune sera obtained by immunizing animals with receptors either under a totally or partially unfolded form (solubilized in 0.1% SDS) or in a rocky state under unfolded aggregated form (dialyzed against water and lyophilized) exhibited a similar antibody activity towards receptors as assessed by western-blot, confocal microscopy and cytofluorometry.

## Claims

1. A method for producing an anti-G Protein-Coupled Receptor (GPCR) antibody, or a cell producing an anti-GPCR antibody, comprising a step of recovering the antibody, or the cell producing the antibody, from a biological sample obtained from an animal which has been immunized with an immunogenic composition prepared from an unfolded GPCR.

2. The method according to claim 1, wherein the unfolded GPCR is obtained by contacting the GPCR composition with a detergent.

3. The method according to claim 2, wherein the detergent is an anionic detergent.

4. The method according to claim 2 or 3, wherein the detergent is sodium dodecyl sulfate (SDS).

5. The method according to any of claims 2 to 4, wherein the unfolded GPCR is obtained by further contacting a GPCR composition with a protein denaturant.

6. The method according to claim 5, wherein the protein denaturant is selected from the group consisting of urea and guanidinium salts.

7. The method according to any of claims 1 to 6, wherein the GPCR composition is produced by a recombinant GPCR-expressing microbial strain.

8. The method according to claim 7, wherein the microbial strain is a yeast strain.

9. The method according to claim 7 or 8, wherein the microbial strain is a *Pichia pastoris* strain.

10. The method according to any of claims 1 to 9, wherein the immunogenic composition is prepared from lyophilizated unfolded GPCR.

11. The method according to any of claims 1 to 10, wherein the immunogenic composition is prepared from unfolded GPCR mixed with a composition comprising at least one amphiphile compound and/or hydrophobic compound.

12. The method of claim 11, wherein the amphiphile compound is D-dianhydromannitol monooleate.

13. The method of claim 11 or 12, wherein the composition comprising at least one amphiphile compound and/or hydrophobic compound is Freund's adjuvant.

14. An immunogenic composition prepared from an unfolded G Protein-Coupled Receptor (GPCR), for use in the immunization of animals.

15. The use of an unfolded G Protein-Coupled Receptor (GPCR) for the manufacture of an immunogenic composition.
